# EUROPEAN PATENT SPECIFICATION

(11) **EP 4 520 369 B1**
(45) Date of publication and mention of the grant of the patent: **02.09.2026**
(21) Application number: 24198800.5
(22) Date of filing: 06.09.2024
(51) Int. Cl.: A61M 11/00, A61M 15/00

(54) **AEROSOL MEDICAMENT DELIVERY SYSTEM**
AEROSOLMEDIKAMENTENABGABESYSTEM
SYSTÈME D'ADMINISTRATION DE MÉDICAMENT

(30) Priority: 07.09.2023 US 202363536944 P
(43) Date of publication of application: 12.03.2025
(73) Proprietor: DelBio, Inc., Taoyuan City 320023 (TW)
(72) Inventor: MA, I Chen, 320023 TAOYUAN CITY (TW); PAI, Sheng Wen, 320023 TAOYUAN CITY (TW); NIEN, Chi Chang, 320023 TAOYUAN CITY (TW)
(74) Representative: Murgitroyd & Company

(56) References cited:
- EP-A1- 3 666 315
- EP-A1- 4 015 094
- US-A1- 2017 319 796
- US-A1- 2023 189 894

## Description

### FIELD OF INVENTION

The present disclosure relates to an aerosol medicament delivery system.

### BACKGROUND OF INVENTION

In recent years, aerosol medicament delivery systems have been widely used in medical care. Nebulizers are a type of aerosol medicament delivery system that can nebulize inhalation liquid-type liquid medicaments into gas to improve the convenience of inhalation treatment for patients.

EP3666315A1 discloses an aerosol delivery device comprising an aerosol generator for generating an aerosol in the aerosol delivery device; a fluid reservoir for receiving the fluid to be aerosolized; a controller which is configured to operate the vibrator, by supplying an electrical drive signal to the vibrator, so as to vibrate the fluid; a phase detector which is configured to detect a phase shift of the drive signal; and an evaluation unit which is configured to detect the presence of fluid in contact with the membrane and/or in the fluid reservoir on the basis of the phase shift detected by the phase detector; wherein the aerosol generator comprises a membrane and a vibrator which is configured to vibrate a fluid and to aerosolize the fluid by the membrane; wherein the fluid reservoir is arranged in fluid communication with the membrane. US2017319796A1 discloses a droplet delivery device including a housing, a reservoir, and ejector mechanism, and at least one differential pressure sensor, wherein the droplet delivery device is automatically breath actuated by a user when a differential pressure sensor senses a predetermined pressure change within the housing, and is then actuated to generate a stream of droplets, so as to target the pulmonary system of the user.

The flow sensing technology of dual pressure sensors is used in conventional nebulizers. The two pressure sensors are arranged inside the flow channel of the nebulizer to achieve differential pressure calculation, and the output signal of the nebulizer is a flow signal, but the disadvantage is that it is more expensive than a nebulizer using a single pressure sensor. In addition, there are also nebulizers using a single pressure sensor. However, when the sensing value of the single pressure sensor deviates in the environment, or the pressure value cannot regress normally, inaccuracy and failure may be caused, resulting in a waste of a medicament and a low dosage rate. Furthermore, the nebulizers with conventional single pressure sensor and flow sensor are expensive and large in size, requiring more space to be placed in the nebulizer.

Therefore, how to provide a nebulizer that is low in cost, easy to carry, does not cause a waste of the medicament and can increase the dosage rate has become an urgent problem that needs to be solved.

### SUMMARY OF INVENTION

In view of the above, the primary object of the present disclosure is to provide an aerosol medicament delivery system, including:
a first pressure sensor configured to detect external air pressure outside the aerosol medicament delivery system;
a second pressure sensor configured to detect air pressure in an airway of the aerosol medicament delivery system;
a controller;
a driver module;
a nebulization module including a mesh, wherein the controller controls the driver module to drive the nebulization module for performing nebulization based on air pressure difference between the external air pressure and the air pressure in the airway;
a detection module configured to detect pressure applied by a medicament onto the mesh, convert the pressure into a voltage signal or a current signal, and transmit the voltage signal or the current signal to the controller, wherein, when the nebulization module (105) is being operated, the controller determines the liquid level of the medicament based on said voltage or current signal, and determines whether the medicament is exhausted based on a change of the voltage signal or the current signal; and
a power supply module configured to provide power for the aerosol medicament delivery system.

In some embodiments, the controller stops the nebulization under a condition that the medicament is exhausted; and continues the nebulization under a condition that the medicament is not yet exhausted.

In some embodiments, the controller determines that the medicament is exhausted under a condition that a slope of the voltage signal or the current signal corresponding to time is greater than a threshold

In some embodiments, the controller adjusts the driver module to reduce an operating voltage or an operating current of the nebulization module, or increase or reduce an operating frequency of the nebulization module after the nebulization module is operated for a predetermined time.

In some embodiments, the controller detects a first operating frequency, a second operating frequency and a third operating frequency of the driver module after the aerosol medicament delivery system is activated;
wherein the controller controls the driver module to drive the nebulization module to nebulize the medicament at the first operating frequency;
wherein the controller controls the driver module to drive the nebulization module to nebulize the medicament at the second operating frequency after the nebulization module is operated for the predetermined time, thereby avoiding overheating of the medicament and causing failure of the medicament.

In some embodiments, a current value corresponding to the first operating frequency is highest, a current value corresponding to the second operating frequency is second highest, and a current value corresponding to the third operating frequency is lower than the current value corresponding to the second operating frequency.

In some embodiments, the third operating frequency is not used for nebulization.

In some embodiments, the detection module (106) provides the voltage signal or the current signal fed back from the nebulization module under the condition that the nebulization module is being operated;
the controller determines a liquid level of the medicament by the controller based on a change of the voltage signal or the current signal; and
the controller adjusts the driver module to reduce a voltage, reduce a current, or increase or reduce a frequency under a condition that the liquid level of the medicament is lower than a predetermined value, thereby avoiding overheating of the medicament and causing failure of the medicament.

In some embodiment, the controller controls the driver module to drive the nebulization module to nebulize the medicament at the second operating frequency under the condition that the liquid level of the medicament is lower than the predetermined value, thereby avoiding overheating of the medicament and causing failure of the medicament.

In some embodiments, the controller detects a respiratory traction pressure of the aerosol medicament delivery system through the first pressure sensor and the second pressure sensor, detects several inhalation cycles and exhalation cycles under a condition that the respiratory traction pressure exceeds a threshold to calculate an average time of the inhalation cycles and an average time of the exhalation cycles, and multiplies the average time of the inhalation cycles and the average time of the exhalation cycles by a set ratio respectively as an adjusted average time of the inhalation cycles and an adjusted average time of the exhalation cycles;
the controller determines whether a user is currently in the inhalation cycles or the exhalation cycles based on a pressure difference detected by the first pressure sensor and the second pressure sensor;
during the inhalation cycles, the controller stops nebulization to reduce waste of medicament under a condition that inhalation time of the user exceeds the adjusted average time of the inhalation cycles, and continues nebulization under a condition that the inhalation time of the user does not exceed the adjusted average time of the inhalation cycles;
during the exhalation cycle, the controller performs nebulization in advance to compensate insufficiency of the medicament caused by a delay under a condition that exhalation time of the user exceeds the adjusted average time of the exhalation cycles, and stopping nebulization under a condition that the exhalation time of the user does not exceed the adjusted average time of the exhalation cycles; and
the controller updates the average time of the inhalation cycles, the average time of the exhalation cycles, the adjusted average time of the inhalation cycles and the adjusted average time of the exhalation cycles after the inhalation cycles and the exhalation cycles end.

In some embodiments, the controller controls the driver module to drive the mesh of the nebulization module at several different operating frequencies after the aerosol medicament delivery system (10) is activated, and detects voltage signals and current signals fed back from the mesh corresponding to the several different operating frequencies through a detection module;
the controller determines the mesh of the nebulization module is operated normally under a condition that several different voltage signals or current signals fed back from the mesh corresponding to the several different operating frequencies are detected by the detection module; and
the controller determines the mesh of the nebulization module is operated abnormally under a condition that consistent voltage signals or current signals fed back from the mesh corresponding to the several different operating frequencies are detected by the detection module.

In some embodiments, the voltage signals and current signals fed back from the mesh corresponding to the several different operating frequencies are not affected by the dosage of the medicament.

In some embodiments, the set ratio is 80% of the average time of the inhalation cycles.

In some embodiments, before S401, the controller performs calibration of the first pressure sensor and the second pressure sensor before the user inhales and exhales.

In some embodiments, under a condition that the air pressure difference between the external air pressure and the air pressure in the airway is greater than a threshold, the controller (103) drives the nebulization module (105) to perform nebulization; and wherein the threshold is 60 Pa

Compared with prior art, the advantages of the aerosol medicament delivery system of the present disclosure are as follows:
(1) small size and low costs;
(2) the voltage or current can be adjusted automatically after a specific period of time, or the voltage or current can be adjusted through a change of the liquid level of the medicament fed back from the mesh to reduce the waste caused by overheating of the medicament;
(3) dual pressure sensors are used to calibrate each other to prevent one of the pressure sensors from being out of accuracy and unable to regress normally, causing abnormal nebulization; and
(4) the time to start nebulization in advance and stop nebulization in advance can be adjusted dynamically based on the user's inhalation cycles and exhalation cycles to avoid unnecessary loss of the medicament.

### DESCRIPTION OF DRAWINGS

For a more complete understanding of the embodiments and their advantages, reference is now made to the following description taken in conjunction with the accompanying drawings, in which:
FIG. 1 shows the composition of an aerosol medicament delivery system 10 of the present disclosure;
FIG. 2 is a flow chart for detecting liquid level of a medicament in aerosol medicament delivery system 10 of the present disclosure;
FIG. 3 shows the relationship between a voltage signal and a current signal fed back from the medicament and nebulization module 105;
FIG. 4 is a flow chart for driving power attenuation of aerosol medicament delivery system 10 according to an embodiment of the present disclosure;
FIGS. 5A and 5B respectively show driving levels of aerosol medicament delivery system 10 of the present disclosure after a voltage or a current of driver module 104 is reduced after a specific time, and the nebulization effect of aerosol medicament delivery system 10 of the present disclosure when an operating frequency of driver module 104 is adjusted after a specific time;
FIG. 6 is a flow chart for driving power attenuation of aerosol drug delivery system 10 according to another embodiment of the present disclosure;
FIGS. 7A and 7B are respectively a schematic diagram of various liquid levels of the medicament in nebulization module 105, and the feedback voltage signals and feedback current signals of various liquid levels of the medicament in nebulization module 105 on a reaction force of the mesh;
FIG. 8 is a flow chart for controlling nebulization of aerosol medicament delivery system 10 of the present disclosure;
FIG. 9 is a graph showing that nebulization module 105 starts nebulization in advance and stops nebulization in advance through a pressure difference between a measurement value of the first pressure sensor and a measurement value of the second pressure sensor;
FIGS. 10A and 10B respectively show the pressure calibration situation of a conventional nebulizer using a single pressure sensor and the pressure calibration situation of aerosol medicament delivery system 10 of the present disclosure using dual pressure sensors; and
FIGS. 11A and 11B respectively show that the nebulization module in normal operation reflects different current signals at different operating frequencies, and the nebulization module in abnormal operation reflects consistent current signals at different operating frequencies.

### DETAILED DESCRIPTION OF PREFERRED EMBODIMENTS

Some specific embodiments according to the present disclosure will be described below; however, the present disclosure can still be practiced in various forms, and the scope of the present disclosure should not be construed as being limited to what is stated in the specification. In addition, unless otherwise indicated in the context, "a", "the" and similar terms used in the specification (especially in the appended claims) should be understood to include both the singular and the plural forms.

Although a series of operations or steps are used below to illustrate the method disclosed herein, the order shown in these operations or steps should not be construed as a limitation of the present disclosure. For example, certain operations or steps may be performed in a different order and/or concurrently with other steps. Furthermore, not all operations, steps, and/or features must be performed to practice embodiments of the present disclosure. Additionally, each operation or step described herein may include several sub-steps or actions.

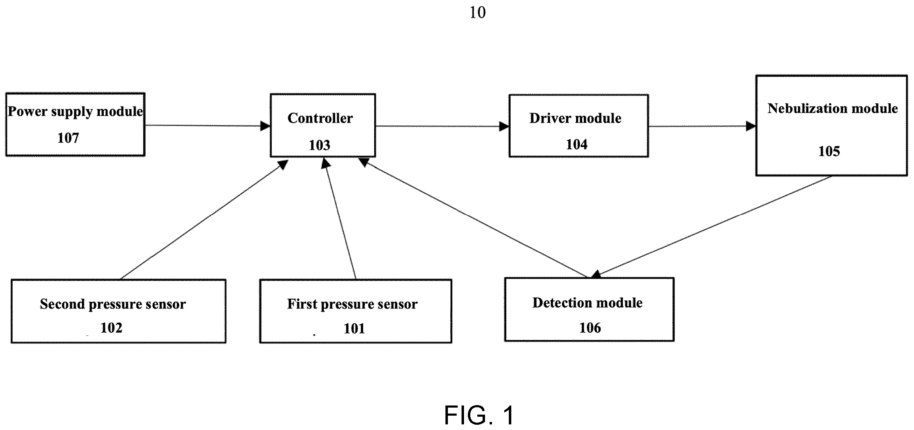

Referring to FIG. 1 first, one embodiment of the present disclosure provides an aerosol medicament delivery system 10, including a first pressure sensor 101, a second pressure sensor 102, a controller 103, a driver module 104, a nebulization module 105, a detection module 106 and a power supply module 107. First pressure sensor 101 is configured to detect external air pressure outside the aerosol medicament delivery system 10. Second pressure sensor 102 is configured to detect air pressure in an airway of aerosol medicament delivery system 10. Nebulization module 105 includes a mesh, and controller 103 controls driver module 104 to drive nebulization module 105 for performing nebulization based on air pressure difference between the external air pressure and the air pressure in the airway. Detection module 106 is configured to detect pressure applied by a medicament onto the mesh, convert the pressure into a voltage signal or a current signal, and transmit the voltage signal or the current signal to controller 103. Controller 103 determines the liquid level of the medicament based on the voltage signal or the current signal. Power supply module 107 is configured to provide power for aerosol medicament delivery system 10.

Specifically, nebulization module 105 connects to the positive and negative electrodes, and detection module 106 electrically connects to nebulization module 105 through the positive and negative electrodes. When nebulization module 105 is activated, the nebulized medicament causes a reaction force, and detection module 106 captures the feedback voltage signal or current signal of nebulization module 105 through the positive and negative electrodes. When the medicament is almost to be exhausted, the force reduces, causing the feedback voltage signal or current signal of nebulization module 105 to increase.

In a specific embodiment, when the air pressure difference between the external air pressure and the air pressure in the airway is greater than a first threshold of 60 Pa, controller 103 drives the nebulization module 105 to perform nebulization.

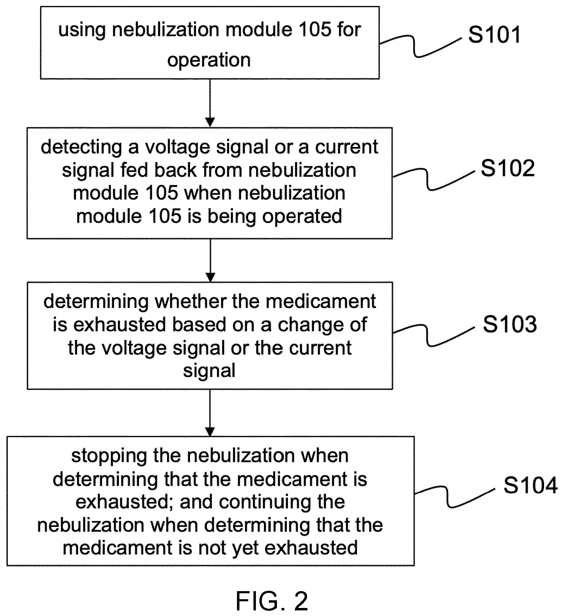

Referring to FIG. 2, the present disclosure provides a method for detecting liquid level of a medicament in aerosol medicament delivery system 10, including steps of:
S101: using nebulization module 105 for operation;
S102: detecting a voltage signal or a current signal fed back from nebulization module 105 when nebulization module 105 is being operated; and
S103: determining whether the medicament is exhausted based on a change of the voltage signal or the current signal.

In a specific embodiment, after S103, the method may further include S104: stopping the nebulization when determining that the medicament is exhausted; and continuing the nebulization when determining that the medicament is not yet exhausted. In a specific embodiment, S103 may further include determining that the medicament is exhausted when a slope of the voltage signal or the current signal corresponding to time is greater than a threshold (e.g., 1).

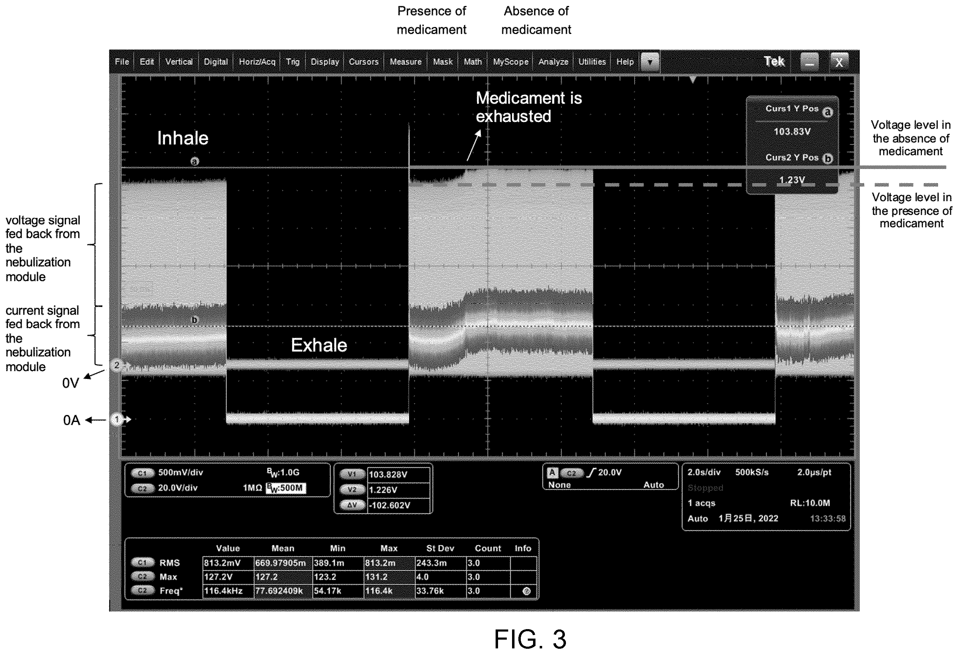

Specifically, FIG. 3 shows the relationship between a voltage signal and a current signal fed back from the medicament and nebulization module 105, and nebulization module 105 is operated at a fixed operating frequency. As shown in FIG.3, when nebulization module 105 is operated, detection module 106 continuously obtains the feedback voltage signal and current signal from nebulization module 105. If there is an offset in the voltage signal and/or current signal, controller 103 captures the feedback voltage signal and/or the current signal to perform a slope calculation. When the slope of the voltage or current versus time is higher than 1, controller 103 determines that the medicament is almost to be exhausted, and further instructs nebulization module 105 to stop being operated or shut down. When the slope is lower than 1, controller 103 determines that the medicament is not yet exhausted and instructs nebulization module 105 to continue operation. The voltage signal and current signal fed back from nebulization module 105 do not cause an erroneous determination of aerosol medicament delivery system 10 due to the pause in exhalation, and the state is continued. Therefore, the present disclosure confirms whether the medicament is present through the difference between the feedback voltage signal and current signal, and detects and controls nebulization module 105 of aerosol medicament delivery system 10 through the feedback voltage signal and current signal.

The present disclosure provides various control methods for aerosol medicament delivery system 10, including driving power attenuation mechanisms of aerosol medicament delivery system 10, which will be described below with specific examples.

### Example 1: driving power attenuation with time

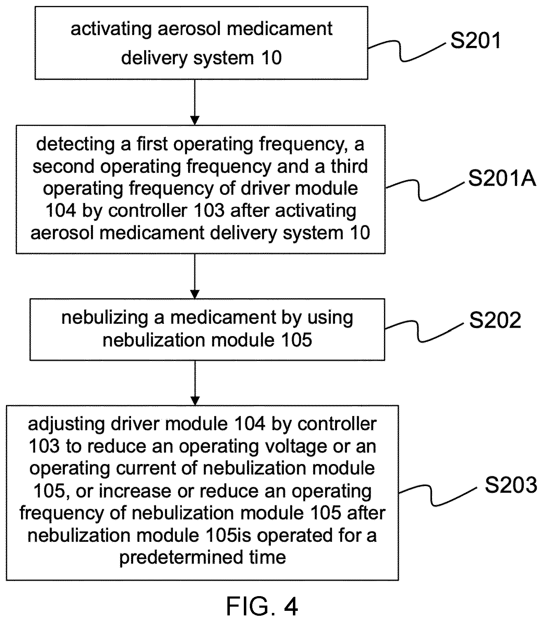

It is known that the nebulizer is driven by a constant power when operating. When the medicament is nebulized for a period of time, the power driven to the nebulization module does not decrease as the amount of the medicament decreases. Thus, the heat energy of the nebulization module may cause overheating and failure of the remaining medicament. In order to reduce the waste caused by overheating of the medicament, as shown in FIG. 4, the present disclosure provides a method for driving power attenuation of aerosol medicament delivery system 10 including controller 103 and driver module 104 configured to drive nebulization module 105 for operation, and the method includes steps of:
S201: activating aerosol medicament delivery system 10;
S202: nebulizing the medicament by using nebulization module105; and
S203: adjusting driver module 104 by controller 103 to reduce an operating voltage or an operating current of nebulization module 105, or increase or reduce an operating frequency of nebulization module 105 after nebulization module 105 is operated for a predetermined time.

In a specific embodiment, after S201 and before S202, the method may further include S201A: detecting a first operating frequency, a second operating frequency and a third operating frequency of driver module 104 by controller 103 after activating aerosol medicament delivery system 10. Preferably, S202 may further include controlling driver module 104 to drive nebulization module 105 by controller 103 to nebulize the medicament at the first operating frequency. Preferably, S203 may further include controlling driver module 104 to drive nebulization module 105 by controller 103 to nebulize the medicament at the second operating frequency after nebulization module 105 is operated for the predetermined time, thereby avoiding overheating of the medicament and causing failure of the medicament.

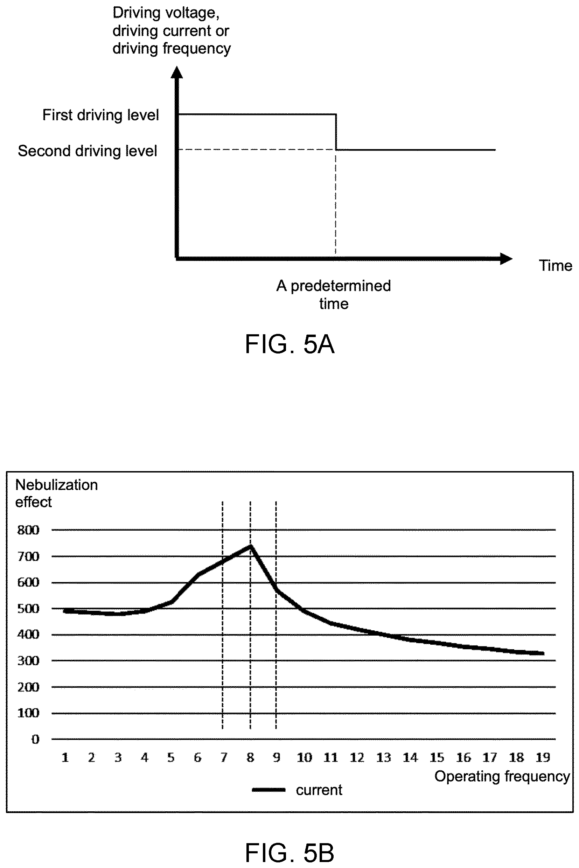

Specifically, the driving power attenuation mechanism includes that aerosol medicament delivery system 10 nebulizes all the medicament when the amount of the medicament is 0.1 ml to 0.3 ml; and since there is a built-in timer in aerosol medicament delivery system 10, the voltage or current of driver module 104 is reduced after a predetermined time (preferably 2 to 3 seconds) to reduce an output power, as shown in FIG. 5A. In addition, the operating frequency may also be switched to a high frequency or a low frequency after a predetermined time (preferably 2 to 3 seconds) to reduce the output power. As shown in FIG. 5B, the current signal is used as the basis for determining the operating frequency, and different frequencies are provided to obtain the feedback current signal of nebulization module 105. Controller 103 defines the highest current signal as the first operating frequency (i.e., the best operating point; "8" corresponding to the horizontal axis shown in FIG. 5B), when controller 103 adjusts the operating frequency after a specific time, because the current signal corresponding to the second operating frequency (i.e., the second best operating point; "7" corresponding to the horizontal axis shown in FIG. 5B) is higher than that corresponding to the third operating frequency ("9" corresponding to the horizontal axis shown in FIG. 5B). Controller 103 automatically selects the second operating frequency for driving power attenuation, so as to avoid heating and failure of the medicament caused by nebulization module 105 when the medicament is reduced. In general, since the nebulization effect of the third operating frequency is very weak, users may have the illusion that there is no nebulization, the third operating frequency is not used. The execution of the aforementioned driving power attenuation mechanism may be completed before aerosol medicament delivery system 10 is activated.

### Example 2: driving power attenuation through detecting a liquid level of a medicament

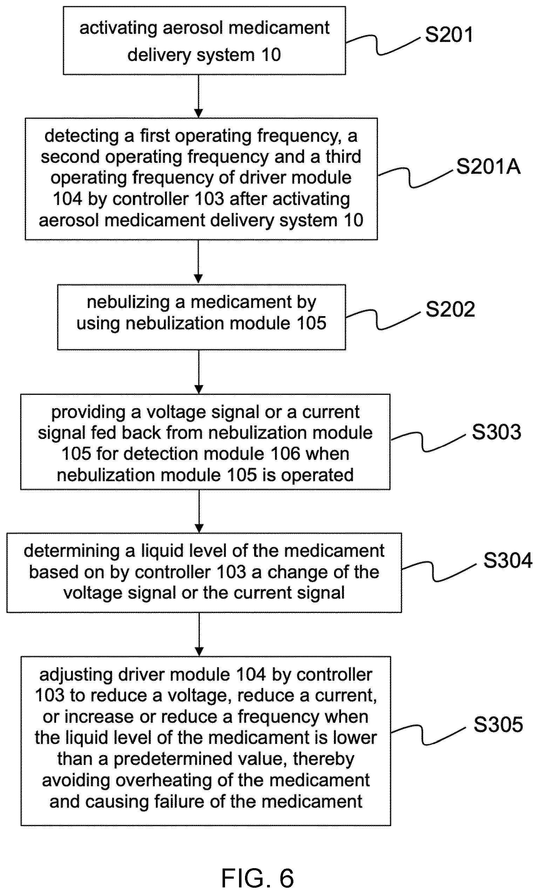

The nebulization of a conventional nebulizer is driven by a constant power. When the nebulizer filled with the medicament nebulizes after a period of time, the power driven to the nebulization module does not decrease as the amount of the medicament decreases. Thus, the heat energy of the nebulization module may cause failure of the remaining medicament. In order to reduce the waste caused by overheating of the medicament, as shown in FIG. 6, the present disclosure provides a method for driving power attenuation of aerosol medicament delivery system 10 further including detection module 106, and the method further includes steps of:
S303: providing a voltage signal or a current signal fed back from nebulization module 105 for detection module 106 when nebulization module 105 is operated;
S304: determining a liquid level of the medicament by controller 103 based on a change of the voltage signal or the current signal; and
S305: adjusting driver module 104 by controller 103 to reduce a voltage, reduce a current, or increase or reduce a frequency when the liquid level of the medicament is lower than a predetermined value, thereby avoiding overheating of the medicament and causing failure of the medicament.

Preferably, S305 may further include controlling driver module 104 to drive nebulization module 105 by controller 103 to nebulize the medicament at the second operating frequency when the liquid level of the medicament is lower than the predetermined value, thereby avoiding overheating of the medicament and causing failure of the medicament.

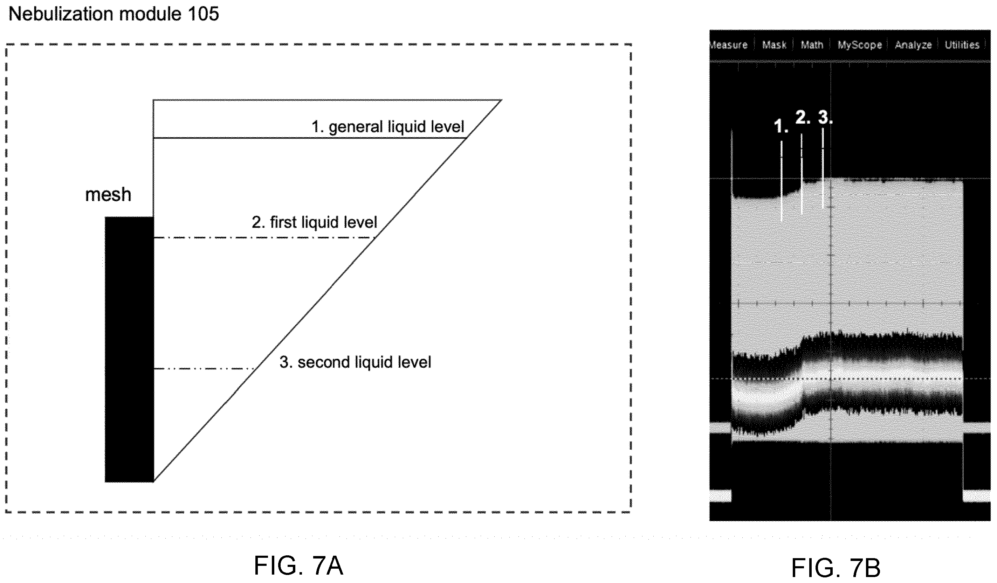

Specifically, FIG. 7A shows that a difference in the reaction forces of the three liquid levels of the medicament applied on the mesh may produce different feedback signals. The general liquid level has a greater reaction force applied on the mesh than the first liquid level, and the first liquid level has a greater reaction force on the mesh than the second liquid level. The aforementioned difference in reaction force may be reflected in the voltage and current of nebulization module 105. As shown in FIG. 7B, the feedback voltage and current signals before the medicament is almost to be exhausted show a slow-rising section. The aforementioned changes in voltage and current signals are used to confirm the liquid level of the medicament, and driving power attenuation is achieved after 1 to 2 seconds, so as to avoid failure of the medicament due to excessive energy of nebulization module 105 and overheating of the remaining medicament. The execution of the aforementioned driving power attenuation mechanism may also be completed before aerosol medicament delivery system 10 is activated.

### Example 3: dosing control of aerosol medicament delivery system

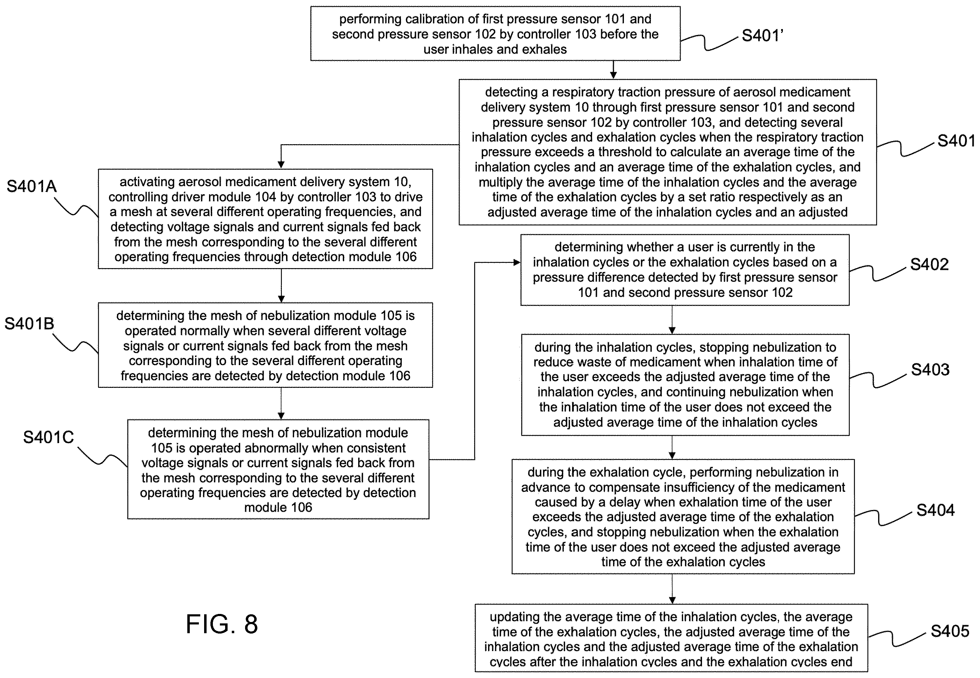

In order to improve the medicament delivery rate of aerosol drug delivery system 10, as shown in FIG. 8, one embodiment of the present disclosure provides a method for controlling nebulization of aerosol medicament delivery system 10, including steps of:
S401: detecting a respiratory traction pressure of aerosol medicament delivery system 10 through first pressure sensor 101 and second pressure sensor 102 by controller 103, and
   detecting several inhalation cycles and exhalation cycles when the respiratory traction pressure exceeds a threshold to calculate an average time of the inhalation cycles and an average time of the exhalation cycles, and multiply the average time of the inhalation cycles and the average time of the exhalation cycles by a set ratio respectively as an adjusted average time of the inhalation cycles and an adjusted average time of the exhalation cycles;
S402: determining whether a user is currently in the inhalation cycles or the exhalation cycles based on a pressure difference detected by first pressure sensor 101 and second pressure sensor 102;
S403: during the inhalation cycles, stopping nebulization to reduce waste of medicament when inhalation time of the user exceeds the adjusted average time of the inhalation cycles, and
   continuing nebulization when the inhalation time of the user does not exceed the adjusted average time of the inhalation cycles;
S404: during the exhalation cycle, performing nebulization in advance to compensate insufficiency of the medicament caused by a delay when exhalation time of the user exceeds the adjusted average time of the exhalation cycles, and
   stopping nebulization when the exhalation time of the user does not exceed the adjusted average time of the exhalation cycles; and
S405: updating the average time of the inhalation cycles, the average time of the exhalation cycles, the adjusted average time of the inhalation cycles and the adjusted average time of the exhalation cycles after the inhalation cycles and the exhalation cycles end.

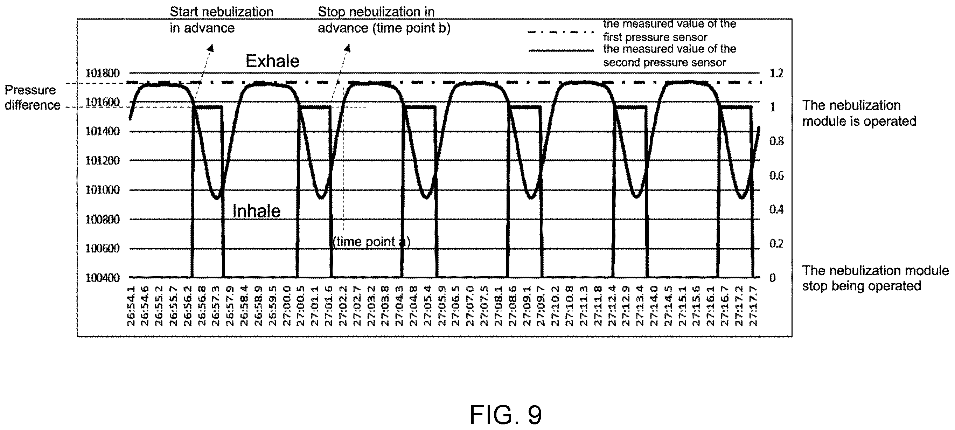

For example, as shown in FIG. 9, if the pressure difference between the measurement value of first pressure sensor 101 minus the measurement value of second pressure sensor 102 is greater than the set threshold (e.g., 60 Pa), it is determined that an inhalation cycle is about to enter, and controller 103 causes the aerosol drug delivery system 10 to start nebulization in advance. If the pressure difference between the measurement value of first pressure sensor 101 minus the measurement value of second pressure sensor 102 is lower than the set threshold (e.g., 60 Pa), it is determined that an exhalation cycle is about to enter (time point a shown in FIG. 9), and controller 103 causes the aerosol drug delivery system 10 to stop nebulization in advance (time point b shown in FIG. 9).

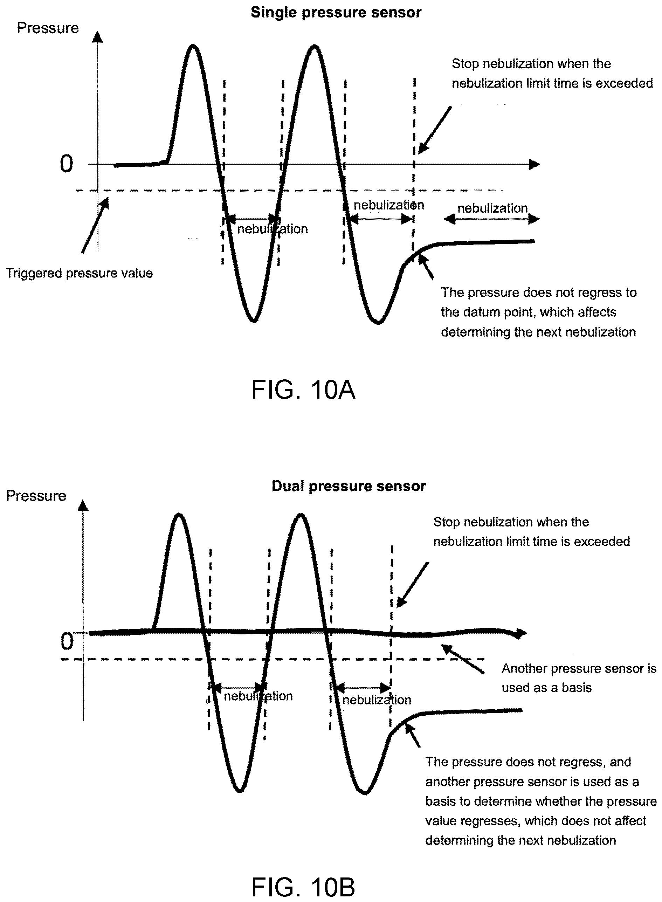

Compared with conventional pressure traction nebulizers, which uses a single pressure, differential pressure or flow sensor for nebulization, a single pressure sensor is initially triggered until the pressure reaches a threshold before subsequent actions can be activated. However, when the single pressure sensor deviates in the environment, or the pressure cannot regress normally, it will cause inaccuracy and failure (as shown in FIG. 10A). The present disclosure uses dual pressure sensors that are calibrated before use, for detecting pressure changes through the second pressure sensor is detected, when the above situation occurs in the second pressure sensor, calibrating the second pressure sensor based on the first pressure sensor, and when the first pressure sensor is out of accuracy, calibrating the first pressure sensor through the second pressure sensor regression on the contrary (as shown in FIG. 10B).

Referring back to FIG. 8, preferably before S401, the method may further include S401': performing calibration of first pressure sensor 101 and second pressure sensor 102 by controller 103 before the user inhales and exhales.

### Example 4: failure detection of the nebulization module

After long-term use of the aerosol medicament delivery system, since the nebulization module has been steamed with wet heat for several times, the piezoelectric element in the nebulization module may gradually separate from the mesh, so that the reaction force of the mesh cannot be transmitted back to the detection module of the aerosol medicament delivery system, resulting in a decrease in the nebulization rate. In order to solve the aforementioned problems, the present disclosure confirms whether the failure of the nebulization module occurs through the calculation of the feedback signal of the nebulization module.

Referring to FIG. 8 again, before S401, the method may further include steps of:
S401A: activating aerosol medicament delivery system10,
   controlling driver module 104 by controller 103 to drive a mesh at several different operating frequencies, and
   detecting voltage signals and current signals fed back from the mesh corresponding to the several different operating frequencies through a detection module;
S401B: determining the mesh of nebulization module 105 is operated normally when several different voltage signals or current signals fed back from the mesh corresponding to the several different operating frequencies are detected by detection module 106; and
S401C: determining the mesh of nebulization module 105 is operated abnormally when consistent voltage signals or current signals fed back from the mesh corresponding to the several different operating frequencies are detected by detection module 106.

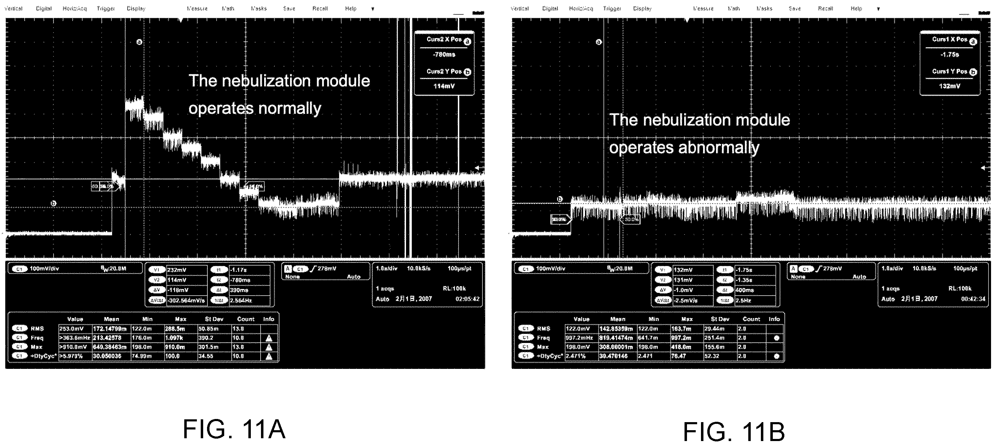

Specifically, when aerosol medicament delivery system 10 is turned on for the first time and operates at different operating frequencies, nebulization module 105 including the mesh and the piezoelectric element may feedback different voltage signals and current signals. Taking FIG. 11 as an example, which shows a current signal. As shown in FIG. 11A, a nebulization module in normal operation reflects different current signals at different operating frequencies. However, as shown in FIG. 11B, a nebulization module in abnormal operation may not reflect different current signals at different operating frequencies due to the damage of the body material. None of the above conditions will be affected by the presence or absence of the medicament.

Given the above, the advantages of the present disclosure are as follows:
(1) small size and low costs;
(2) the voltage or current can be adjusted automatically after a specific period of time, or the voltage or current can be adjusted through a change of the liquid level of the medicament fed back from the mesh to reduce the waste caused by overheating of the medicament;
(3) dual pressure sensors are used to calibrate each other to prevent one of the pressure sensors from being out of accuracy and unable to regress normally, causing abnormal nebulization; and
(4) the time to start nebulization in advance and stop nebulization in advance can be adjusted dynamically based on the user's inhalation cycles and exhalation cycles to avoid unnecessary loss of the medicament.

## Claims

1. An aerosol medicament delivery system (10), wherein the system comprises:
a first pressure sensor (101) configured to detect external air pressure outside the aerosol medicament delivery system (10);
a second pressure sensor (102) configured to detect air pressure in an airway of the aerosol medicament delivery system (10);
a controller (103);
a driver module (104);
a nebulization module (105) comprising a mesh, wherein the controller (103) controls the driver module (104) to drive the nebulization module (105) for performing nebulization based on air pressure difference between the external air pressure and the air pressure in the airway;
a detection module (106) configured to detect pressure applied by a medicament onto the mesh, convert the pressure into a voltage signal or a current signal, and transmit the voltage signal or the current signal to the controller (103), wherein, when the nebulization module (105) is being operated, the controller (103) determines the liquid level of the medicament based on said voltage or current signal, and determines whether the medicament is exhausted based on a change of the voltage signal or the current signal; and
a power supply module (107) configured to provide power for the aerosol medicament delivery system (10).

2. The aerosol medicament delivery system (10) of claim 1, wherein:
the controller (103) stops the nebulization under a condition that the medicament is exhausted; and continues the nebulization under a condition that the medicament is not yet exhausted;
wherein the controller (103) determines that the medicament is exhausted under a condition that a slope of the voltage signal or the current signal corresponding to time is greater than a threshold.

3. The aerosol medicament delivery system (10) of claim 1, wherein: the controller (103) adjusts the driver module (104) to reduce an operating voltage or an operating current of the nebulization module (105), or increase or reduce an operating frequency of the nebulization module (105) after the nebulization module (105) is operated for a predetermined time.

4. The aerosol medicament delivery system (10) of claim 3, wherein:
the controller (103) detects a first operating frequency, a second operating frequency and a third operating frequency of the driver module (104) after the aerosol medicament delivery system (10) is activated;
wherein the controller (103) controls the driver module (104) to drive the nebulization module (105) to nebulize the medicament at the first operating frequency;
wherein the controller (103) controls the driver module (104) to drive the nebulization module (105) to nebulize the medicament at the second operating frequency after the nebulization module is operated for the predetermined time, thereby avoiding overheating of the medicament and causing failure of the medicament.

5. The aerosol medicament delivery system (10) of claim 3 or 4, wherein:
the detection module (106) provides the voltage signal or the current signal fed back from the nebulization module (105) under the condition that the nebulization module (105) is being operated;
the controller (103) determines a liquid level of the medicament based on a change of the voltage signal or the current signal; and adjusts the driver module (104) to reduce a voltage, reduce a current, or increase or reduce a frequency under a condition that the liquid level of the medicament is lower than a predetermined value, thereby avoiding overheating of the medicament and causing failure of the medicament.

6. The aerosol medicament delivery system (10) of claim 4 or 5 when depending on claim 4, wherein the controller (103) controls the driver module (104) to drive the nebulization module (105) to nebulize the medicament at the second operating frequency under the condition that the liquid level of the medicament is lower than the predetermined value, thereby avoiding overheating of the medicament and causing failure of the medicament.

7. The aerosol medicament delivery system (10) of claim 6, wherein a current value corresponding to the first operating frequency is highest, a current value corresponding to the second operating frequency is second highest, and a current value corresponding to the third operating frequency is lower than the current value corresponding to the second operating frequency.

8. The aerosol medicament delivery system (10) of claim 6, wherein the third operating frequency is not used for nebulization.

9. The aerosol medicament delivery system (10) of claim 1, wherein:
the controller (103) detects a respiratory traction pressure of the aerosol medicament delivery system (10) through the first pressure sensor (101) and the second pressure sensor (102), detects several inhalation cycles and exhalation cycles in a condition that the respiratory traction pressure exceeds a threshold to calculate an average time of the inhalation cycles and an average time of the exhalation cycles, and multiplies the average time of the inhalation cycles and the average time of the exhalation cycles by a set ratio respectively as an adjusted average time of the inhalation cycles and an adjusted average time of the exhalation cycles;
the controller (103) determines whether a user is currently in the inhalation cycles or the exhalation cycles based on a pressure difference detected by the first pressure sensor (101) and the second pressure sensor (102);
during the inhalation cycles, the controller (103) stops nebulization to reduce waste of medicament under a condition that inhalation time of the user exceeds the adjusted average time of the inhalation cycles, and continues nebulization under a condition that the inhalation time of the user does not exceed the adjusted average time of the inhalation cycles;
during the exhalation cycle, the controller (103) performs nebulization in advance to compensate insufficiency of the medicament caused by a delay under a condition that exhalation time of the user exceeds the adjusted average time of the exhalation cycles, and stops nebulization under a condition that the exhalation time of the user does not exceed the adjusted average time of the exhalation cycles; and
the controller (103) updates the average time of the inhalation cycles, the average time of the exhalation cycles, the adjusted average time of the inhalation cycles and the adjusted average time of the exhalation cycles after the inhalation cycles and the exhalation cycles end.

10. The aerosol medicament delivery system (10) of claim 1 wherein
the controller (103) controls the driver module (104) to drive the mesh of the nebulization module at several different operating frequencies after the aerosol medicament delivery system (10) is activated, and detects voltage signals and current signals fed back from the mesh corresponding to the several different operating frequencies through a detection module (106); the controller (103) determines the mesh of the nebulization module (105) is operated normally under a condition that several different voltage signals or current signals fed back from the mesh corresponding to the several different operating frequencies are detected by the detection module (106); and
the controller (103) determines the mesh of the nebulization module (105) is operated abnormally under a condition that consistent voltage signals or current signals fed back from the mesh corresponding to the several different operating frequencies are detected by the detection module (106).

11. The aerosol medicament delivery system (10) of claim 10, wherein the voltage signals and current signals fed back from the mesh corresponding to the several different operating frequencies are not affected by the dosage of the medicament.

12. The aerosol medicament delivery system (10) of claim 9, wherein the set ratio is 80% of the average time of the inhalation cycles.

13. The aerosol medicament delivery system (10) of claim 9, wherein the controller (103) performs calibration of the first pressure sensor (101) and the second pressure sensor (102) before the user inhales and exhales.

14. The aerosol medicament delivery system (10) of claim 1, wherein under a condition that the air pressure difference between the external air pressure and the air pressure in the airway is greater than a threshold, the controller (103) drives the nebulization module (105) to perform nebulization; and wherein the threshold is 60 Pa

## Patentansprüche

1. Ein Aerosolarzneimittelabgabesystem (10), wobei das System Folgendes beinhaltet:
einen ersten Drucksensor (101), der konfiguriert ist, um einen externen Luftdruck außerhalb des Aerosolarzneimittelabgabesystems (10) zu erfassen;
einen zweiten Drucksensor (102), der konfiguriert ist, um einen Luftdruck in einem Atemweg des Aerosolarzneimittelabgabesystems (10) zu erfassen;
eine Steuerung (103);
ein Treibermodul (104);
ein Vernebelungsmodul (105), das ein Netz beinhaltet, wobei die Steuerung (103) das Treibermodul (104) steuert, um das Vernebelungsmodul (105) zum Durchführen einer Vernebelung basierend auf einer Luftdruckdifferenz zwischen dem externen Luftdruck und dem Luftdruck in dem Atemweg anzutreiben;
ein Erfassungsmodul (106), das konfiguriert ist, um einen durch ein Arzneimittel auf das Netz ausgeübten Druck zu erfassen, den Druck in ein Spannungssignal oder ein Stromsignal umzuwandeln und das Spannungssignal oder das Stromsignal an die Steuerung (103) zu übertragen, wobei, wenn das Vernebelungsmodul (105) betrieben wird, die Steuerung (103) den Flüssigkeitsstand des Arzneimittels basierend auf dem Spannungs- oder Stromsignal bestimmt, und basierend auf einer Änderung des Spannungssignals oder des Stromsignals bestimmt, ob das Arzneimittel erschöpft ist; und
ein Stromversorgungsmodul (107), das konfiguriert ist, um Strom für das Aerosolarzneimittelabgabesystem (10) bereitzustellen.

2. Aerosolarzneimittelabgabesystem (10) gemäß Anspruch 1, wobei:
die Steuerung (103) die Vernebelung unter einer Bedingung stoppt, dass das Arzneimittel erschöpft ist; und die Vernebelung unter einer Bedingung fortsetzt, dass das Arzneimittel noch nicht erschöpft ist;
wobei die Steuerung (103) unter einer Bedingung bestimmt, dass das Arzneimittel erschöpft ist, dass eine Steigung des Spannungssignals oder des Stromsignals, die der Zeit entspricht, größer als ein Schwellenwert ist.

3. Aerosolarzneimittelabgabesystem (10) gemäß Anspruch 1, wobei:
die Steuerung (103) das Treibermodul (104) anpasst, um eine Betriebsspannung oder einen Betriebsstrom des Vernebelungsmoduls (105) zu reduzieren oder eine Betriebsfrequenz des Vernebelungsmoduls (105) zu erhöhen oder zu reduzieren, nachdem das Vernebelungsmodul (105) für eine vorbestimmte Zeit betrieben wurde.

4. Aerosolarzneimittelabgabesystem (10) gemäß Anspruch 3, wobei:
die Steuerung (103) eine erste Betriebsfrequenz, eine zweite Betriebsfrequenz und eine dritte Betriebsfrequenz des Treibermoduls (104) erfasst, nachdem das Aerosolarzneimittelabgabesystem (10) aktiviert wurde;
wobei die Steuerung (103) das Treibermodul (104) steuert, um das Vernebelungsmodul (105) anzutreiben, um das Arzneimittel bei der ersten Betriebsfrequenz zu vernebeln;
wobei die Steuerung (103) das Treibermodul (104) steuert, um das Vernebelungsmodul (105) anzutreiben, um das Arzneimittel bei der zweiten Betriebsfrequenz zu vernebeln, nachdem das Vernebelungsmodul für die vorbestimmte Zeit betrieben wurde, wodurch eine Überhitzung des Arzneimittels vermieden wird und ein Ausfall des Arzneimittels verursacht wird.

5. Aerosolarzneimittelabgabesystem (10) gemäß Anspruch 3 oder 4, wobei:
das Erfassungsmodul (106) das Spannungssignal oder das Stromsignal bereitstellt, das von dem Vernebelungsmodul (105) unter der Bedingung rückgekoppelt wird, dass das Vernebelungsmodul (105) betrieben wird;
die Steuerung (103) einen Flüssigkeitsstand des Arzneimittels basierend auf einer Änderung des Spannungssignals oder des Stromsignals bestimmt; und das Treibermodul (104) anpasst, um eine Spannung zu reduzieren, einen Strom zu reduzieren oder eine Frequenz zu erhöhen oder zu reduzieren, unter einer Bedingung, dass der Flüssigkeitsstand des Arzneimittels niedriger als ein vorbestimmter Wert ist, wodurch eine Überhitzung des Arzneimittels vermieden wird und ein Ausfall des Arzneimittels verursacht wird.

6. Aerosolarzneimittelabgabesystem (10) gemäß Anspruch 4 oder 5 in Abhängigkeit von Anspruch 4, wobei die Steuerung (103) das Treibermodul (104) steuert, um das Vernebelungsmodul (105) anzutreiben, um das Arzneimittel bei der zweiten Betriebsfrequenz zu vernebeln, unter der Bedingung, dass der Flüssigkeitsstand des Arzneimittels niedriger als der vorbestimmte Wert ist, wodurch eine Überhitzung des Arzneimittels vermieden wird und ein Ausfall des Arzneimittels verursacht wird.

7. Aerosolarzneimittelabgabesystem (10) gemäß Anspruch 6, wobei ein Stromwert, der der ersten Betriebsfrequenz entspricht, am höchsten ist, ein Stromwert, der der zweiten Betriebsfrequenz entspricht, am zweithöchsten ist, und ein Stromwert, der der dritten Betriebsfrequenz entspricht, niedriger als der Stromwert ist, der der zweiten Betriebsfrequenz entspricht.

8. Aerosolarzneimittelabgabesystem (10) gemäß Anspruch 6, wobei die dritte Betriebsfrequenz nicht zur Vernebelung verwendet wird.

9. Aerosolarzneimittelabgabesystem (10) gemäß Anspruch 1, wobei:
die Steuerung (103) einen Atemzugdruck des Aerosolarzneimittelabgabesystems (10) durch den ersten Drucksensor (101) und den zweiten Drucksensor (102) erfasst, mehrere Inhalationszyklen und Ausatmungszyklen unter einer Bedingung erfasst, dass der Atemzugdruck einen Schwellenwert überschreitet, um eine durchschnittliche Zeit der Inhalationszyklen und eine durchschnittliche Zeit der Ausatmungszyklen zu berechnen, und die durchschnittliche Zeit der Inhalationszyklen und die durchschnittliche Zeit der Ausatmungszyklen mit einem festgelegten Verhältnis als eine angepasste durchschnittliche Zeit der Inhalationszyklen bzw. eine angepasste durchschnittliche Zeit der Ausatmungszyklen multipliziert;
die Steuerung (103) bestimmt, ob sich ein Benutzer derzeit in den Inhalationszyklen oder den Ausatmungszyklen befindet, basierend auf einer Druckdifferenz, die durch den ersten Drucksensor (101) und den zweiten Drucksensor (102) erfasst wird;
während der Inhalationszyklen die Steuerung (103) die Vernebelung stoppt, um Arzneimittelverschwendung zu reduzieren, unter einer Bedingung, dass die Inhalationszeit des Benutzers die angepasste durchschnittliche Zeit der Inhalationszyklen überschreitet, und die Vernebelung unter einer Bedingung fortsetzt, dass die Inhalationszeit des Benutzers die angepasste durchschnittliche Zeit der Inhalationszyklen nicht überschreitet;
während des Ausatmungszyklus die Steuerung (103) eine Vernebelung im Voraus durchführt, um eine Insuffizienz des Arzneimittels zu kompensieren, die durch eine Verzögerung verursacht wird, unter einer Bedingung, dass die Ausatmungszeit des Benutzers die angepasste durchschnittliche Zeit der Ausatmungszyklen überschreitet, und die Vernebelung unter einer Bedingung stoppt, dass die Ausatmungszeit des Benutzers die angepasste durchschnittliche Zeit der Ausatmungszyklen nicht überschreitet; und
die Steuerung (103) die durchschnittliche Zeit der Inhalationszyklen, die durchschnittliche Zeit der Ausatmungszyklen, die angepasste durchschnittliche Zeit der Inhalationszyklen und die angepasste durchschnittliche Zeit der Ausatmungszyklen aktualisiert, nachdem die Inhalationszyklen und die Ausatmungszyklen enden.

10. Aerosolarzneimittelabgabesystem (10) gemäß Anspruch 1, wobei
die Steuerung (103) das Treibermodul (104) steuert, um das Netz des Vernebelungsmoduls bei mehreren unterschiedlichen Betriebsfrequenzen anzutreiben, nachdem das Aerosolarzneimittelabgabesystem (10) aktiviert wurde, und Spannungssignale und Stromsignale erfasst, die von dem Netz entsprechend den mehreren unterschiedlichen Betriebsfrequenzen durch ein Erfassungsmodul (106) rückgekoppelt werden;
die Steuerung (103) bestimmt, dass das Netz des Vernebelungsmoduls (105) normal betrieben wird, unter einer Bedingung, dass mehrere unterschiedliche Spannungssignale oder Stromsignale, die von dem Netz entsprechend den mehreren unterschiedlichen Betriebsfrequenzen rückgekoppelt werden, durch das Erfassungsmodul (106) erfasst werden; und
die Steuerung (103) bestimmt, dass das Netz des Vernebelungsmoduls (105) anormal betrieben wird, unter einer Bedingung, dass konsistente Spannungssignale oder Stromsignale, die von dem Netz entsprechend den mehreren unterschiedlichen Betriebsfrequenzen rückgekoppelt werden, durch das Erfassungsmodul (106) erfasst werden.

11. Aerosolarzneimittelabgabesystem (10) gemäß Anspruch 10, wobei die Spannungssignale und Stromsignale, die von dem Netz entsprechend den mehreren unterschiedlichen Betriebsfrequenzen rückgekoppelt werden, nicht durch die Dosierung des Arzneimittels beeinflusst werden.

12. Aerosolarzneimittelabgabesystem (10) gemäß Anspruch 9, wobei das festgelegte Verhältnis 80 % der durchschnittlichen Zeit der Inhalationszyklen beträgt.

13. Aerosolarzneimittelabgabesystem (10) gemäß Anspruch 9, wobei:
die Steuerung (103) eine Kalibrierung des ersten Drucksensors (101) und des zweiten Drucksensors (102) durchführt, bevor der Benutzer inhaliert und ausatmet.

14. Aerosolarzneimittelabgabesystem (10) gemäß Anspruch 1, wobei
unter einer Bedingung, dass die Luftdruckdifferenz zwischen dem externen Luftdruck und dem Luftdruck in dem Atemweg größer als ein Schwellenwert ist, die Steuerung (103) das Vernebelungsmodul (105) antreibt, um eine Vernebelung durchzuführen; und wobei der Schwellenwert 60 Pa beträgt.

## Revendications

1. Un système d'administration de médicament sous forme d'aérosol (10), où le système comprend :
un premier capteur de pression (101) configuré afin de détecter une pression d'air externe à l'extérieur du système d'administration de médicament sous forme d'aérosol (10) ;
un deuxième capteur de pression (102) configuré afin de détecter une pression d'air dans une voie d'air du système d'administration de médicament sous forme d'aérosol (10) ;
un dispositif de commande (103) ;
un module pilote (104) ;
un module de nébulisation (105) comprenant un maillage, où le dispositif de commande (103) commande le module pilote (104) afin de piloter le module de nébulisation (105) pour effectuer une nébulisation sur la base d'une différence de pression d'air entre la pression d'air externe et la pression d'air dans la voie d'air ;
un module de détection (106) configuré afin de détecter une pression appliquée par un médicament sur le maillage, de convertir la pression en un signal de tension ou un signal de courant, et de transmettre le signal de tension ou le signal de courant au dispositif de commande (103), où, lorsque le module de nébulisation (105) est mis en fonctionnement, le dispositif de commande (103) détermine le niveau de liquide du médicament sur la base dudit signal de tension ou de courant, et détermine si le médicament est épuisé sur la base d'un changement du signal de tension ou du signal de courant ; et
un module d'alimentation en puissance (107) configuré afin de fournir de la puissance pour le système d'administration de médicament sous forme d'aérosol (10).

2. Le système d'administration de médicament sous forme d'aérosol (10) de la revendication 1, où :
le dispositif de commande (103) arrête la nébulisation dans une condition où le médicament est épuisé ; et continue la nébulisation dans une condition où le médicament n'est pas encore épuisé ;
où le dispositif de commande (103) détermine que le médicament est épuisé dans une condition où une pente du signal de tension ou du signal de courant correspondant au temps est supérieure à un seuil.

3. Le système d'administration de médicament sous forme d'aérosol (10) de la revendication 1, où :
le dispositif de commande (103) ajuste le module pilote (104) afin de réduire une tension de fonctionnement ou un courant de fonctionnement du module de nébulisation (105), ou d'augmenter ou de réduire une fréquence de fonctionnement du module de nébulisation (105) après le fonctionnement du module de nébulisation (105) pendant un temps prédéterminé.

4. Le système d'administration de médicament sous forme d'aérosol (10) de la revendication 3, où :
le dispositif de commande (103) détecte une première fréquence de fonctionnement, une deuxième fréquence de fonctionnement et une troisième fréquence de fonctionnement du module pilote (104) après l'activation du système d'administration de médicament sous forme d'aérosol (10) ;
où le dispositif de commande (103) commande le module pilote (104) afin de piloter le module de nébulisation (105) pour nébuliser le médicament à la première fréquence de fonctionnement ;
où le dispositif de commande (103) commande le module pilote (104) afin de piloter le module de nébulisation (105) pour nébuliser le médicament à la deuxième fréquence de fonctionnement après le fonctionnement du module de nébulisation pendant le temps prédéterminé, évitant de ce fait une surchauffe du médicament et de causer une défaillance du médicament.

5. Le système d'administration de médicament sous forme d'aérosol (10) de la revendication 3 ou de la revendication 4, où :
le module de détection (106) fournit le signal de tension ou le signal de courant renvoyé depuis le module de nébulisation (105) dans la condition où le module de nébulisation (105) est mis en fonctionnement ;
le dispositif de commande (103) détermine un niveau de liquide du médicament sur la base d'un changement du signal de tension ou du signal de courant ; et ajuste le module pilote (104) afin de réduire une tension, de réduire un courant, ou d'augmenter ou de réduire une fréquence dans une condition où le niveau de liquide du médicament est inférieur à une valeur prédéterminée, évitant de ce fait une surchauffe du médicament et de causer une défaillance du médicament.

6. Le système d'administration de médicament sous forme d'aérosol (10) de la revendication 4 ou de la revendication 5 lorsqu'elle dépend de la revendication 4, où le dispositif de commande (103) commande le module pilote (104) afin de piloter le module de nébulisation (105) pour nébuliser le médicament à la deuxième fréquence de fonctionnement dans la condition où le niveau de liquide du médicament est inférieur à la valeur prédéterminée, évitant de ce fait une surchauffe du médicament et de causer une défaillance du médicament.

7. Le système d'administration de médicament sous forme d'aérosol (10) de la revendication 6, où une valeur de courant correspondant à la première fréquence de fonctionnement est la plus élevée, une valeur de courant correspondant à la deuxième fréquence de fonctionnement est la deuxième plus élevée, et une valeur de courant correspondant à la troisième fréquence de fonctionnement est inférieure à la valeur de courant correspondant à la deuxième fréquence de fonctionnement.

8. Le système d'administration de médicament sous forme d'aérosol (10) de la revendication 6, où la troisième fréquence de fonctionnement n'est pas utilisée pour la nébulisation.

9. Le système d'administration de médicament sous forme d'aérosol (10) de la revendication 1, où :
le dispositif de commande (103) détecte une pression de traction respiratoire du système d'administration de médicament sous forme d'aérosol (10) par l'intermédiaire du premier capteur de pression (101) et du deuxième capteur de pression (102), détecte plusieurs cycles d'inspiration et cycles d'expiration dans une condition où la pression de traction respiratoire dépasse un seuil afin de calculer un temps moyen des cycles d'inspiration et un temps moyen des cycles d'expiration, et multiplie le temps moyen des cycles d'inspiration et le temps moyen des cycles d'expiration par un rapport défini respectivement en tant que temps moyen ajusté des cycles d'inspiration et temps moyen ajusté des cycles d'expiration ;
le dispositif de commande (103) détermine si un utilisateur est actuellement dans les cycles d'inspiration ou les cycles d'expiration sur la base d'une différence de pression détectée par le premier capteur de pression (101) et le deuxième capteur de pression (102) ;
pendant les cycles d'inspiration, le dispositif de commande (103) arrête la nébulisation afin de réduire le gaspillage de médicament dans une condition où le temps d'inspiration de l'utilisateur dépasse le temps moyen ajusté des cycles d'inspiration, et continue la nébulisation dans une condition où le temps d'inspiration de l'utilisateur ne dépasse pas le temps moyen ajusté des cycles d'inspiration ;
pendant le cycle d'expiration, le dispositif de commande (103) effectue une nébulisation à l'avance afin de compenser une insuffisance du médicament causée par un retard dans une condition où le temps d'expiration de l'utilisateur dépasse le temps moyen ajusté des cycles d'expiration, et arrête la nébulisation dans une condition où le temps d'expiration de l'utilisateur ne dépasse pas le temps moyen ajusté des cycles d'expiration ; et
le dispositif de commande (103) met à jour le temps moyen des cycles d'inspiration, le temps moyen des cycles d'expiration, le temps moyen ajusté des cycles d'inspiration et le temps moyen ajusté des cycles d'expiration après la fin des cycles d'inspiration et des cycles d'expiration.

10. Le système d'administration de médicament sous forme d'aérosol (10) de la revendication 1, où
le dispositif de commande (103) commande le module pilote (104) afin de piloter le maillage du module de nébulisation à plusieurs fréquences de fonctionnement différentes après l'activation du système d'administration de médicament sous forme d'aérosol (10), et détecte des signaux de tension et des signaux de courant renvoyés depuis le maillage correspondant aux plusieurs fréquences de fonctionnement différentes par l'intermédiaire d'un module de détection (106) ;
le dispositif de commande (103) détermine que le maillage du module de nébulisation (105) est en fonctionnement normal dans une condition où plusieurs signaux de tension ou signaux de courant différents renvoyés depuis le maillage correspondant aux plusieurs fréquences de fonctionnement différentes sont détectés par le module de détection (106) ; et
le dispositif de commande (103) détermine que le maillage du module de nébulisation (105) est en fonctionnement anormal dans une condition où des signaux de tension ou signaux de courant cohérents renvoyés depuis le maillage correspondant aux plusieurs fréquences de fonctionnement différentes sont détectés par le module de détection (106).

11. Le système d'administration de médicament sous forme d'aérosol (10) de la revendication 10, où les signaux de tension et signaux de courant renvoyés depuis le maillage correspondant aux plusieurs fréquences de fonctionnement différentes ne sont pas affectés par le dosage du médicament.

12. Le système d'administration de médicament sous forme d'aérosol (10) de la revendication 9, où le rapport défini est 80 % du temps moyen des cycles d'inspiration.

13. Le système d'administration de médicament sous forme d'aérosol (10) de la revendication 9, où :
le dispositif de commande (103) effectue un étalonnage du premier capteur de pression (101) et du deuxième capteur de pression (102) avant que l'utilisateur n'inspire et n'expire.

14. Le système d'administration de médicament sous forme d'aérosol (10) de la revendication 1, où
dans une condition où la différence de pression d'air entre la pression d'air externe et la pression d'air dans la voie d'air est supérieure à un seuil, le dispositif de commande (103) pilote le module de nébulisation (105) pour effectuer une nébulisation ; et où le seuil est de 60 Pa.
